# EUROPEAN PATENT SPECIFICATION

(11) **EP 2 049 097 B1**
(45) Date of publication and mention of the grant of the patent: **04.11.2020**
(21) Application number: 07730774.2
(22) Date of filing: 22.05.2007
(51) Int. Cl.: A61K 31/198, A61P 39/00, A61P 1/00, A61P 1/04, A61K 9/00

(54) **COMPOSITION AND METHOD FOR BINDING ACETALDEHYDE IN STOMACH**
ZUBEREITUNG UND METHODE ZUM BINDEN VON ACETALDEHYD IM MAGEN
COMPOSITION ET MÉTHODE DE LIAISON DE L'ACÉTALDÉHYDE DANS L'ESTOMAC

(30) Priority: 22.05.2006 US 802120 P; 22.05.2006 FI 20060501
(43) Date of publication of application: 22.04.2009
(73) Proprietor: BIOHIT OYJ, 00880 Helsinki (FI)
(72) Inventor: SUOVANIEMI, Osmo, FI-00570 Helsinki (FI); SALASPURO, Ville, FI-00029 HUS (FI); MARVOLA, Martti, FI-00660 Helsinki (FI); SALASPURO, Mikko, FI-00260 Helsinki (FI)
(74) Representative: Laine IP Oy
(86) International application number: PCT/FI2007/050287
(87) International publication number: WO 2007/135241

(56) References cited:
- WO-A-02/36098
- WO-A-2006/103316
- US-B2- 6 696 262
- SALASPURO ET AL.: "Binding of ethanol derived carcinogenic acetaldehyde in colonic contents by slow releasing L-cysteine tablet" ALCOHOLISM CLINICAL AND EXPERIMENTAL RESEARCH, vol. 26, no. 5suppl., May 2002 (2002-05), - May 2002 (2002-05) page 139A, XP009088965 SCIENTIFIC MEETING OF THE RESEARCH SOCIETY ON ALCOHOLISM AND THE 11TH CONGRESS OF THE INTERNATIONAL; SAN FRANCISCO, CALIFORNIA, USA
- SALASPURO V ET AL: "REMOVAL OF ACETALDEHYDE FROM SALIVA BY A SLOW-RELEASE BUCCAL TABLET OF L-CYSTEINE" INTERNATIONAL JOURNAL OF CANCER, NEW YORK, NY, US, vol. 97, 2002, pages 361-364, XP002908015 ISSN: 0020-7136 cited in the application
- SALASPURO ET AL.: "Eliminating Carcinogenic Acetaldehyde by Cysteine From Saliva During Smoking" CANCER EPIDEMIOÖOGY, BIOMARKERS & PREVENTION, vol. 15, no. 1, January 2006 (2006-01), pages 146-149, XP002449219 USA cited in the application

## Description

This invention relates to a composition for binding acetaldehyde in the stomach or in the stomach, intestine and/or colon. This invention relates also to decreasing the risk of developing cancer in the gastrointestinal tract caused by acetaldehyde.

Both alcohol and smoking are risk factors for upper digestive tract cancers, and the combined use thereof multiplies the risk of developing an upper digestive tract cancer to as much as 150-fold (Salaspuro, M. Best Pract Res Clin. Gastroenterol (2003) 17:679 - 94 and Francheschi et al. Cancer Res (1990) 50:6502 - 07).

The first metabolite of alcohol is acetaldehyde. It has been shown to be carcinogenic both to test animals and humans (Salaspuro, M. Crit Rev Clin Lab Sci (2003) 40: 183 - 208). Alcohol is evenly distributed in the liquid phase of the organs. Hence, after enjoying alcohol and as long as there is alcohol in the organs, the alcohol content in blood, saliva, gastric juice and the contents of the intestine is the same. In that case, the microbes in the digestive tract are capable of oxidizing the alcohol to acetaldehyde. For example, even after a moderate dose of ethanol (0.5 g/kg), high acetaldehyde contents of a microbial origin (18 - 143 µM) have been found in human saliva; in other words, acetaldehyde builds up in saliva as an intermediate product of the microbial metabolism (Homann et al, Carcinogenesis (1997) 18:1739 - 1743). During active smoking, the acetaldehyde in saliva was increased to a value of 261.4±45.5 µM from the basic level (Salaspuro et al. (2004) Int J Cancer, 2004 Sep 10; 111(4):480 - 3).

Asian heavy drinkers, who have a familial low-activity modification of the aldehyde dehydrogenase-2 (ALDH2) enzyme, have both an increased risk of developing a cancer of the mouth, the pharynx and the digestive tract, and an increased acetaldehyde content of the saliva after consuming alcohol (Väkeväinen et al. (2000) Alcohol Clin Exp Res 24:873 - 877). Even more common is the ADH3^{∗}1 gene/allele (ADH1C at present), which predisposes the heavy drinkers, who have this gene, to the upper digestive tract cancers because of increased local acetaldehyde contents. (Visapää J-P et al. Gut. 2004 Jun; 53(6):871-6.)

In the organism, acetaldehyde is thus formed from alcohol as a consequence of the hepatic metabolism and, locally, in the digestive tract via microbial alcohol dehydrogenase (Salaspuro et al, (1996) Ann Med 28:195 - 200). The average amount of saliva secreted by a human is 1.5 litres per day. The areas of influence of the acetaldehyde contained in the saliva include the mouth, the pharynx, the oesophagus and the stomach. Consequently, the effects of acetaldehyde may extend to the whole upper digestive tract area. On the other hand, carcinogenic acetaldehyde can be produced also endogenously by the oral microbes from various foodstuffs with high sugar or carbohydrate content, especially in an achlorhydric stomach. Atrophic gastritis and achlorhydria are well known risk factors of gastric cancer.

As a consequence of the microbial metabolism, acetaldehyde builds up in the stomach in the case, where the stomach is free from acid or has been made acid-free by medication Väkeväinen et al. (2000) Alimentary Pharmacology Ther 14:1511 - 1518) describes an experiment, where the pH of stomach fluid was raised from pH 1.3 to 6.1. Volunteers were given ethanol (0.6 g/kg as 15 vol % solution). After 40 minutes incubation there were in the gastric juice 0.7 to 4.1 % alcohol and 30 µM to 100 µM acetaldehyde. The acetaldehyde content of the gastric juice was the higher the more there were bacteria in the stomach. In the gastric juice there were for example *Streptococcus viridans-* bacteria, which have been shown to be excellent producers of acetaldehyde. Other effective acetaldehyde producers in acid-free stomach have been shown to be bacteria belonging to *Neisseria, Rothia* and *Streptococcus salivarius* (Väkeväinen (2002) et al. Scand J Gastroenterol 37:648-655).

For atrophic gastritis patients, microbes produce high acetaldehyde contents from ethanol and sugar in the stomach leading to an enhanced gastric cancer risk among atrophic gastritis patients (Väkeväinen et al, Scand J Gastroenterol 2002 (6): 648 - 655). In the experiments of Väkeväinen et al. sugar (3 ml/kg, 10 w-% glucose) or ethanol (0.3 g/kg; 15 vol-%) were infused to stomach. After sugar infusion and 60 minutes incubation three patients from 16 had 2.3 to 13.3 µM endogenous acetaldehyde and 2.3 to 13.3 uM ethanol in their stomach. After alcohol infusion the average amount of acetaldehyde was 44.5 µM which is 6.5 times more than what controls had.

Our recent studies show that in an achlorhydric stomach alcohol fermentation can start very quickly by the bacteria representing normal flora of the mouth or by yeasts present in the foodstuffs, for example by common baker's or brewer's yeast. These microbes can produce significant amounts of acetaldehyde and ethanol for example from carbohydrate containing foodstuffs, such as rice. This happens in particular, if the carbohydrate containing foodstuff is sweetened. For example in Asian countries the use of sweet sauces with rice is a very common practise. According to epidemiological studies the eating of rice causes a high risk for cancer in stomach.

In acid stomach the alcohol fermentation does not occur. On the other hand *Helicobacter pylori* infection and certain medicaments, such as Protein Pump Inhibitors (PPI) raise the pH of the stomach.

About nearly 25 % of the human population in the world suffers from atrophic gastritis. From the Finnish population about 8 to 12 % (depending on the age) suffers from atrophic gastritis and the disease is even more common among elder people. The development of achlorhydric stomach is a risk factor also for people having oesophagus reflux disease, if it is treated by PPI medicamends. About 25 % of the human population in the world has this disease.

One further risk factor are foodstuffs comprising acetaldehyde. Our recent studies have shown that all sugar (saccharose, maltose, lactose) containing foodstuffs including beverages, can contain - or in the foodstuff is formed - significant amounts of acetaldehyde, 5 to 2000 µM and ethanol, 0.1 to 0.5 per mille. Some sour milks, yoghurts and juices contain acetaldehyde and ethanol as such (PCT/FI2006/000104).

It has also been shown that acetaldehyde builds up in the large intestine, as its bacteria that represent the normal flora are capable of converting ethanol into acetaldehyde (Jokelainen et al, (1996) Gut 39:100 - 104). In the intestines, endogenous ethanol can also be found, i.e. ethanol that is formed in the intestines in oxygen-free conditions under the effect of microbes. Acetaldehyde is formed, when this ethanol comes into contact with oxygen near the mucous membrane, for example.

The prior art discloses pharmaceutical compositions which contain compounds that bind acetaldehyde, their effect being based on the reaction of the effective substances with the acetaldehyde inside blood and/or cells, for example, US 5 202 354, US 4 496 548, US 4 528 295, US 5 922 346.

Acetaldehyde, which is formed in the organism when alcohol is consumed and thereafter, causes physiological symptoms called a hangover. Previously, efforts have been made to decrease the symptoms caused by acetaldehyde by taking preparations containing ascorbic acid, thiamine, cysteine or cysteic acid, and flavonoids or flavonoid complexes in a form of orally taken tablets in connection with, before or after consuming alcohol. When swallowed, the effective substances go to the stomach and small intestine and from there into the blood circulation (Matsuoka, US Pat No 5,202,354 and Moldowan et al, US Pat No 4,496,548).

Suggestions have been made so as to use preparations containing amino acids, such as L-cysteine, methionine, taurine or arginine, ascorbic acid, vitamins A and E, which are sucked or chewed in the mouth, to reduce the effect of detrimental free radical compounds, which are formed when using tobacco products or being exposed to the same. It is believed that, after being absorbed, amino acids affect various tissues (Hersch, US Pat No 5,922,346, Hersch, International Patent Application WO 99/00106).

Publication WO 02/36098 suggests the use of compounds containing a free sulfhydryl and/or amino group for a local and long-term binding of acetaldehyde from saliva, the stomach or the large intestine. The compounds were mixed with a substance that enabled them to be released for at least 30 minutes in the conditions of the mouth, the stomach or the large intestine.

Publication WO 2006/037848 suggest a composition comprising one or more free suldhydryl and/or amino groups for removing or decreasing the aldehyde content of the saliva during smoking.

As on the basis of our recent studies, acetaldehyde plays a considerable part in the pathogenesis of the stomach cancers, in particular by people having achlorhydric stomach or atrophic gastritis. There is thus a need to find alternative ways to bind acetaldehyde in the stomach and also lower part of digestive tract in a harmless manner.

### Summary

It is an aim of the present invention to provide new compositions, which can be used to reduce the acetaldehyde content in the stomach. It is also an aim of the present invention to provide new compositions, which can be used to reduce the acetaldehyde content of small intestine (called here intestine) and/or large intestine (called here colon).

It is also an aim of the present invention to provide new compositions for binding acetaldehyde in the stomach and/or intestine and/or colon of people having increased risk for cancer in these areas.

It is also an aim of the present invention to provide new compositions for treating people diagnosed to have increased risk for cancer in the stomach and/or intestine and/or colon. In particular, it is an aim of the invention to provide new compositions for treating people having at least one of the biomarkers of atrophic gastritis and/or achlorhydric or low acid stomach.

It is also an aim of the present invention to provide new compositions, which mask the taste of the acetaldehyde-binding compound)(s) in the composition. In particular, it is an aim of the invention to protect the acetaldehyde-binding compound)(s) not to be released too early, i.e. in mouth when they are consumed or if they are mixed with a foodstuff, in the foodstuff.

These and other objects, together with the advantages thereof over known compositions are achieved by the present invention, as hereinafter described and claimed.

One object of the present invention is thus a composition, which comprises one or more acetaldehyde-binding compounds.

According to the invention the composition binds acetaldehyde present in the stomach or in the stomach, intestine and/or colon and comprises one or more acetaldehyde-binding compound(s), which are bound to a non-toxic carrier that effects, in the stomach, sustained release of said compound(s) into the stomach.

To be more precise, the composition according to the invention is characterized in that, what is stated in the characterizing part of claim 1.

The invention provides considerable advantages. The compositions comprising acetaldehyde-binding compounds can be used to reduce the risk of developing the cancer of the stomach, the intestine and/or colon of people having increased risk for cancer in these areas. By the compositions of the invention can be treated in particular people suffering from atrophic gastritis, achlorhydric and low acid stomach. More specifically, by the compositions of the present invention can be treated people having atrophic gastritis, atrophic gastritis of corpus or atrophic gastritis of antrum or achlorhydric or low acid stomach or *Helicobacter pylori* infection. In particular, the compositions according to the invention can be used for decreasing the risk of cancer or for treating people having at least one of the biomarkers of atrophic gastritis. Such biomarkers are low pepsinogen I (PI) level, low pepsinogen I (PI)/pepsinogen II (PII) ratio, high gastrin- 17 level compared to the reference range or cut-off values. Furthermore, the compositions according to the invention can be used for decreasing the risk of cancer or for treating people having at least one of the biomarkers of achlorhydric or low acid stomach. Such biomarkers are high gastrin-17 value, high PI value and high PII value compared to the reference range values. High HPAB (*Helicobacter pylori* antibody) value is a biomarker of atrophic gastritis and achlorhydric and low acid stomach, since it may raise the pH of the stomach. All these biomarkers can be tested by commercially available GastroPanel ®.

Furthermore, the compositions of the present invention are effective for binding acetaldehyde, in particular, when they are consumed in connection of eating, before, during or after eating. The composition is capable of releasing acetaldehyde-binding compounds in stomach during the time the foodstuff is digested. However, the compositions can be used also in a continuous manner, for example after every 8 to 10 hours. The composition may comprise a carrier that does not dissolve in the stomach or comprises a water insoluble film releasing the effective substance only slowly. Alternatively the composition may comprise substances which form a gel in the stomach or which adhere the composition to the mucous membrane of the stomach.

The compositions of the invention may be protected not to be released too early, in mouth, but be released in the stomach. The compositions may be covered or coated by a water-soluble film. This hinders effectively the potentially unpleasant taste of acetaldehyde-binding compound(s). Alternatively, the composition may be in the form of a tablet or capsule, preferably a hard gelatine or HPMC capsule.

Consuming the compositions according to the invention mainly binds acetaldehyde locally, but it may also have a systemic effect.

In addition, the compositions according to the invention can be used for large-scale consumers of alcohol, or those who have hangover, smokers and those, who have a familial low-activity modification of the aldehyde dehydrogenase-2 (ALDH2) enzyme or the ADH3^{∗}1 gene/allele (ADH1C^{∗}1 at present). The use of the compositions according to the invention is also of benefit to those who consume moderate amounts of alcohol or who consume foodstuffs that contain small contents of alcohol or acetaldehyde.

### Detailed description of the invention

### Definitions

A composition comprising acetaldehyde-binding compound(s) means in connection of this invention a composition which comprises a non-toxic carrier(s), which is/are not harmful for human (or animal) consumption. The composition may mean a functional food additive comprising a liquid or solid material intended to be added to a foodstuff or it may mean a product for reducing the risks for diseases. The composition may also mean a pharmaceutical composition comprising pharmaceutically acceptable carriers. The compositions in particular suitable for oral administration. The carriers as such may comprise the same substances and it depends on the legislation of the country, whether the composition should be called a food additive, a product for reducing the risks for diseases or a pharmaceutical composition. The aim of the composition is to decrease the risk for cancer in the gastro-intestinal tract.

The composition comprises an effective amount of acetaldehyde-binding compound(s). An effective amount means an amount capable of binding or inactivating the amount of acetaldehyde present in a foodstuff or formed during the digesting of a foodstuff in the stomach after eating. An effective amount may mean also an amount capable of binding or inactivating the amount of acetaldehyde present in the stomach due to acetaldehyde formed from alcohol or for other reasons in stomach or in intestine and/or colon.

"A composition for binding acetaldehyde present in the stomach" means here a composition, which comprises one or more acetaldehyde-binding compound(s). Preferably said compound(s) comprise one or more free sulphhydryl and/or amino groups, more preferably one or more free sulphhydryl and amino groups.

The composition comprises a non-toxic carrier that effects, in the stomach, sustained release of said compound(s) in the stomach. Sustained or prolonged release means the release of effective substances for at least 30 minutes in the conditions of the stomach. Preferably the effective substances release for 0.5 to 8 hours, preferably 2 to 6 hours, most preferably 2 to 4 hours.

According to a preferred embodiment of the invention the compositions are taken in connection of eating, preferably during the eating, before the eating or after eating. The composition can be for example mixed to the foodstuff or it can be taken before or after eating. The composition preferably releases the effective compound(s) the time the foodstuff is in the stomach i.e. during the digestion of the food. This time is typically 2 to 4 hours.

According to some embodiments of the invention the dosage may be renewed by 4 to 10 hour intervals, preferably at 6 to 8-hour intervals.

The composition according to the invention is in a form of a monolithic or multiparticular preparation, such as tablet or capsule or granule.

A single dose of the preparation may be a tablet or capsule or suitable amount of granules or a tablet or capsule comprising granules or powder.

It is of advantage if the composition is in the form of a preparation, the diameter of which is at least 7 mm, preferably 8 to 15 mm, more preferably 11 to 15 mm. This assists the preparation to stay in the stomach sufficient time for the sustained release of acetaldehyde-binding compound(s).

The amount of compound(s) released in the conditions of the stomach is preferably 40-80 mg in an hour.

The task of the carrier in the composition is sustained release of the effective compound(s) in the conditions of the stomach.

According to one preferred embodiment of the invention the composition comprises a carrier that does not dissolve or dissolves only poorly in the stomach. Alternatively the composition may be covered by a water insoluble film.

According to another embodiment of the invention the carrier may form a gel in the stomach that floats in the contents of the stomach.

According to one further embodiment of the invention the preparation may attach to the mucous membrane of the stomach.

According to one preferred embodiment of the invention the composition comprises a carrier that does not dissolve in the stomach. Such a carrier may be a polymer, such as metacrylate polymer, for example Eudragit RS or S, or ethyl cellulose.

The composition may comprise substances selected from the group comprising one or more acetaldehyde-binding compound(s), a polymer not dissolving in the stomach and a bulking agent.

The composition comprises acetaldehyde-binding compound(s) 1 to 40 w- %, preferably 5 to 40, more preferably 10 to 30 w- %. Typically the amount is 20 to 25 w-%.

The composition comprises polymers 10-50 w-% preferably 20 to 40 w-%, more preferably 20 to 30 w-%.

The composition comprises bulking agents 20-70 w-%, preferably 40 to 60 w-%, most preferably about 50 w-%. Preferably, the bulking agents are selected from calcium hydrogen phosphate and microcrystalline cellulose.

According to one preferred embodiment of the invention the composition comprises matrix granules not dissolving in stomach. The composition may comprise for example:

| | |
|---|---|
| Acetaldehyde binding compound(s) | 5 to 40 w-% (preferably 25 w-% ) |
| Polymer not dissolving in stomach | 10 to 50 w-% (preferably 20 to 30 w-% ) |
| Inert bulking agent | 20 to 70 w-% (preferably 40 to 60 w-% ) |
| Ethanol | q.s. |

The polymer not dissolving in stomach may be any in pharmaceutical industry commonly used additive, such as metacrylate polymer, for example Eudragit RS or S, or ethyl cellulose (EC). The inert bulking agent may be for example calcium hydrogen phosphate, microcrystalline cellulose (MCC), or other corresponding non-swelling agent. The solid substances are mixed and moistured by ethanol. The moisture mixture is granulated by using in pharmaceutical industry well known methods and devices. The dried granules can be used as such or distributed into dosages, for example into capsules.

According to another preferred embodiment of the invention the composition comprises matrix tablets not dissolving in stomach. The composition may comprise for example:

| | |
|---|---|
| Asetaldehyde binding compound(s) | 5 to 40 w-% (preferably 25 w-% ) |
| Polymer not dissolving in stomach | 10 to 50 w-% (preferably 20 to 30 w-% ) |
| Inert bulking agent | 20 to 70 w-% (preferably 20 to 50 w-% ) |

The polymer not dissolving in stomach may be any in pharmaceutical industry commonly used additive, such as metacrylate polymer, for example Eudragit RS or S, or ethyl cellulose (EC). The inert bulking agent may be for example calcium hydrogen phosphate, microcrystalline cellulose (MCC), or other corresponding non-swelling agent. The solid substances are mixed and the mixture is granulated by using for example ethanol or hydrophilic polymer solution. The granules are pressed to tablets by in pharmaceutical industry well known methods and devices. The release of the effective compound(s) is now based on the diffusion of the water- soluble effective compound(s) from the pores formed to the tablet matrix.

According to one preferred embodiment of the invention the composition may be protected in a form so that the compounds are not released in mouth. The granules, tablets and capsules may be covered by a water-soluble film, which effectively covers or masks the taste of acetaldehyde-binding compound(s).

According to another preferred embodiment of the invention the composition may comprise substances selected from the group comprising one or more acetaldehyde-binding compound(s), water-soluble bulking agent(s) and porous film forming agent(s) for coating the preparation.

The composition preferably comprises acetaldehyde-binding compound(s) 1 to 50 w- %, preferably 5 to 40 w- %, more preferably 20 to 50 w- %, still more preferably 20 to 30 w-%. Typically the amount is about 20 to 25 w-%.

The composition preferably comprises bulking agent(s) 10-80 w-% preferably 40 to 80 w-%, more preferably 50 to 60 w-%.

The composition preferably comprises porous film forming agents, such as ethyl cellulose and hydroxypropyl methylcellulose. The relative amount of EC to HPMC may be 3/2 to 7/3.

A comparative preparation, preferably tablet, covered by a film not dissolving in stomach. The composition may comprise for example:

| | |
|---|---|
| Asetaldehyde binding compound(s) | 1 to 50 w-% (preferably 20 to 50 w-%) |
| Water-soluble bulking agent(s) | 50 to 80 w-% (preferably 30 to 60 w-%) |
| Porous film forming agent(s) | q.s. |

The water-soluble bulking agent may be for example lactose or some other in pharmaceutical industry commonly used water-soluble bulking agent. The solid substances are mixed and the mixture is pressed to tablets by in pharmaceutical industry well-known methods and devices. The porous film may be prepared from a water-soluble polymer, such as hydroxypropyl methyl cellulose (HPMC) and water-insoluble polymer, such as ethyl cellulose (EC). The relative amount of the film forming substances, for example EC and HPMC, may be 2-5 parts EC and 1-2 parts HPMC. In the conditions of the stomach the water-soluble polymer dissolves and pores are formed to the water insoluble polymer. The release of the effective compound(s) is now based on the diffusion of the water-soluble effective compound(s) from the pores formed to the film. The film forming substances effectively mask also the taste of acetaldehyde binding compound(s).

Since acetaldehyde is formed also in the large intestine, for example in connection of drinking alcoholic beverages, it is of advantage, if the composition is protected in a form so that the compounds are not released until in the large intestine. Such a protection may be a polymer film that dissolves in an environment with a pH of 6.5 or higher, typically at pH 6.0-7.5, preferably 6.5-7.0.

A film coating, which does not dissolve in the acidic environment of the stomach, but dissolves at a pH value of 7.5 at the latest, can be made both on the tablet or the granules or the capsules. In making the preparation, it is also possible to use polysaccharides that degrade under the effect of microbes of the large intestine, or polymers generated by azo bonds. The form of preparation known by the trade name Oros™ can also be used, when its opening is first covered with an enteric polymer, the solution pH of which is ≈ 7.

Useful enteric polymers include, for example, the grades of hydroxypropyl methylcellulose phthalate, hydroxypropyl methylcellulose succinate or hydroxypropyl methylcellulose-acetatesuccinate (HPMC-AS) or the like, such as sold by the trade name Aqoat™, Aqoat AS-HF™ in particular, a cellulose acetatephtalate (CAP) grade sold by the trade name Aquateric™, and methacrylic acid derivative, methacrylic acid-methylmethacrylate copolymers, the grade sold by the trade name Eudragit-S™ in particular.

The composition according to the invention may have at least one ingredient, which adjusts the release of the effective substance not to take place until at the end of the small intestine or in the large intestine. This component can be a polymer that dissolves depending on the pH (=enteric polymer) or a polymer that degrades under the effect of the enzymes secreted by the bacteria of the large intestine. The polymer that controls the place of release can form a film around the entire preparation. It can also form a film around the particles (granules) contained by the multiple-part preparation. The polymer that degrades under the effect of the enzymes secreted by the bacteria of the large intestine can also be as a filler in a monolithic preparation, or as a filler in the granules or in a multiple-unit preparation prepared from these granules. See also WO 02/36098.

The preparation one embodiment of the invention the preparation may be an enteric tablet, the film coating of which does not dissolve until at the end of the small intestine or at the beginning of the large intestine. The dissolution pH of the polymer that forms the film may be 6.0-7.5, preferably 6.5-7.0. The amount of enteric polymer that forms the film may be 5-20%, preferably 10-15% of the whole mass of the tablet. The filler of the tablet can comprise pharmaceutical additives that do not swell, such as calcium hydrogen phosphate.

The preparation according to the invention can also be granules that comprise an acetaldehyde-binding compound(s) and are coated with an enteric film, the dissolution pH of the film-forming polymer being 6.0-7.5, preferably 6.5-7.0. The amount of film-forming enteric polymer of the entire mass of the granule may be 5-30%, preferably 15-25%. The granule can comprise 20-40%, preferably about 30% of filler poorly soluble in water, such as calcium hydrogen phosphate.

The binder of the granule coated with the enteric film, according to the invention, can be an enteric polymer, the dissolution pH of which is 6.0-7.5, preferably 6.5-7.0. The amount of binder in the granule may be 2-5%, preferably 3-4%.

The preparation according to the invention can also be a tablet comprising the enteric coated granules described above, on which an enteric film has also been made. The tablet made for such a preparation not only comprises enteric granules, but also a filler suitable for direct compression, such as microcrystalline cellulose, the amount of which in the tablet is 30-70%, preferably 40-60%.

The composition of the enteric tablet, which comprises enteric granules and binds acetaldehyde in the desired way, can be as follows, for example:

**Enteric granules:**

| | |
|---|---|
| Acetaldehyde-binding substance | 100 mg |
| Filler, e.g., calcium hydrogen phosphate | 30 - 50 mg |
| Enteric polymers | 40 - 60 mg |

**Enteric tablet:**

| | |
|---|---|
| Enteric granules | 170 - 210 mg |
| Microcrystalline cellulose | 170 - 210 mg |
| Lubricants (e.g. magnesium stearate and talcum) | 5 - 10 mg |
| Enteric polymers | 30 - 50 mg |

Since acetaldehyde is formed also in the small intestine, for example in connection of drinking alcoholic beverages or is carried there from the stomach, it is of advantage, if the composition is protected in a form so that the compounds are not released until in the small intestine. Such a protection may be a polymer, such as Eudragit L, that dissolves in an environment with a pH of pH 5 to 6.

The composition of the present invention can also be in a form of a preparation comprising a fraction for binding acetaldehyde present in the stomach and in addition a fraction protected in a form so that the compounds are not released until in the large intestine. The ratio of the composition for stomach and the composition for the large intestine may be 1:1 to 1:3, typically 1:2.

The composition of the present invention can also be in a form of a preparation comprising a fraction for binding acetaldehyde present in the stomach and in addition a fraction protected in a form so that the compounds are not released until in the small intestine and in addition a fraction protected not released until in the large intestine. The ratio of the composition for stomach and the composition for the small intestine and for the large intestine may 2:1:1 to 1:3:3, typically it may be 2:1:1, 1:1:1, 1:1:2, 1:1:3, 1:2:2,1:2:3, 1:1:3 or 1: 3:3.

The preparation may comprise the substances intended for a preparation for binding acetaldehyde in the stomach. Optionally the preparation may be in the form of a capsule, such as HPMC capsule or gelatine, particularly hard gelatine.

According to another embodiment of the invention the carrier may form a gel in the stomach that floats in the contents of the stomach.

According to one further embodiment of the invention the preparation may be a liquid preparation taken orally (mixture), the physical structure of which is a gel.

According to one further embodiment of the invention the preparation may attach to the mucous membrane of the stomach.

For these embodiments the carrier may be selected from the group comprising various chitosans, alginates, such as sodium alginate, aluminium hydroxide, sodium hydrogen carbonate, sodium carboxymethyl cellulose, and sodium hydrogen carbonate as described in WO 02/36098.

The composition may be monolithic or multiparticular tablet or capsule or granule as such, which, when wetted under the influence of the gastric juices adhere to the mucous membrane of the stomach or form a gel that floats in the contents of the stomach, as a consequence of which their residence time in the stomach is prolonged and thus enables a prolonged release in and a local effect of the drug on the stomach. The long-acting preparation that locally acts on the stomach can be a liquid preparation taken orally (mixture), the physical structure of which is a gel.

A special property required of the pharmaceutical composition that has a local effect on the stomach is that it remains in the stomach for as long time as possible. Technically, this can be solved in two ways: by making a preparation that adheres to the mucous membrane of the stomach or making a preparation that floats in the contents of the stomach. The preparation can be rendered fixable to the mucous membrane of the stomach by using as additives cationic polymers, such as various chitosan grades. Preparations that float in the stomach are provided by using polymers, such as alginic acid, that form a gel and by adding to the preparation sodium hydrogen carbonate, which under the influence of gastric acid releases carbon dioxide, which in turn forms gas bubbles inside the gel.

Comparative examples of compositions that float in the stomach are liquid gels that float in the stomach, which can be prepared from sodium alginate, aluminium hydroxide, sodium hydrogen carbonate, and water, to which the acetaldehyde-binding compound can be added. A corresponding liquid preparation is also obtained by adding an acetaldehyde-binding substance to an aqueous dispersion of chitosan.

Another embodiment of the invention includes a preparation that remains in the stomach for a long time, which is a preparation, which is known as HBS™ (hydrodynamically balanced system). The preparation can remain in the stomach for a long time, when a relatively large tablet is made of it (with a diameter of at least 7-10 mm) and it is coated with a film, which does not decompose in the alimentary tract, and which, however, releases an effective substance (Oros™) through a hole which has been made to it, for example. Preferably such a preparation is consumed before, during or after eating.

When needed, the dosage may be renewed at 4 to 10-hour intervals, preferably at 6 to 8-hour intervals.

The amount of compound released in the conditions of the stomach is preferably 40-80 mg in an hour.

The preparation according to the invention, which releases in the stomach, has at least one - often two - polymers, which have the task of keeping the drug as long time as possible, for two hours minimum, in the stomach either so that it attaches the preparation to the mucous membrane of the stomach or forms a gel that floats in the contents of the stomach. Another task of the polymers is to prolong the release of the effective substance.

The preparation that locally binds acetaldehyde in the stomach can be a tablet that forms a gel in the stomach or a capsule comprising a mixture of powder or granules that forms a gel. In addition to the acetaldehyde-binding substances, the preparation comprises polymers that form a gel in the stomach, such as chitosans, alginates, sodium carboxy-methylcellulose grades, carbomers or aluminium hydroxide. To advance floating in the stomach, the preparation can also comprise sodium hydrogen carbonate.

The amount of polymers in the preparation is 10-50%, preferably 15-40%, and most preferably 20-30%.

The amount of sodium hydrogen carbonate may be 10-30%, preferably 20% of the amount of polymers.

The preparation that locally binds acetaldehyde in the stomach can be a tablet or granule preparation, wherein the acetaldehyde-binding substance is mixed with the fillers needed and, after that, granulated by using enteric polymers as binders. The binder used can be any known enteric polymer, preferably a polymer with a solution pH of 6-7, and most preferably the polymer is any of the methacrylate derivatives, which are known by the trade names Eudragit L and Eudragit S. The amount of enteric polymer in the preparation is preferably 2-5%, most preferably 3-4%.

A comparative example of a preparation that locally binds acetaldehyde in the stomach can be a liquid preparation, i.e., a mixture comprising, in addition to the acetaldehyde-binding substance, also sodium alginate, aluminium hydroxide, sodium hydrogen carbonate, and water. The amount of water in the whole preparation is 70-90%, most preferably about 75-85%. The amount of sodium alginate in the preparation is preferably 2-10%, most preferably about 5%, and the amount of aluminium hydroxide is preferably 5-15%, most preferably about 10%.

The relative composition of a comparative preparation comprising granules can be as follows, for example:

| | | |
|---|---|---|
| Acetaldehyde-binding substances | 60 | parts |
| Chitosan | 10 - 40 | parts |
| Calcium hydrogen phosphate | 0 - 30 | parts |

The relative composition of the comparative liquid preparation can be as follows, for example:

| | | |
|---|---|---|
| Acetaldehyde-binding substances | 10 | parts |
| Sodium alginate | 2 - 10 | parts |
| Aluminium hydroxide | 5 - 15 | parts |
| Sodium hydrogen carbonate | 1 - 2 | parts |
| Water | 70 - 80 | parts |

An "acetaldehyde-binding compound(s)" refers to a compound(s) containing one or more free sulphhydryl and/or amino groups, preferably one or more sulphhydryl and amino group(s), most preferably in close proximity to each other (1,2- or 1,3 disubstituted aminothiols). Instead of sulphhydryl group may be used sulphone group. "Compound" may be used to refer to one or more compounds. Also compounds comprising one or more SH-group(s) and/or one or more amino group(s) function in suitable concentrations.

The "binding of acetaldehyde" preferably refers to a chemical reaction between the acetaldehyde and the compound that has a free sulphhydryl and/or amino group, wherein the acetaldehyde jointly with the "acetaldehyde-binding substance" forms a larger molecule and water can be formed in the reaction. For example, when reacting with cysteine, the acetaldehyde binds itself both to the sulphhydryl and the amino group and forms 2-methyl-L-thiazolidine-4-carboxylic acid and water. The acetaldehyde can bind itself to the amino group of almost any protein, whereby Schiffs base or a 2-methylimidazole ring is formed.

According to the invention, the compounds obtained from acetaldehyde by chemically binding are safe for the organism.

It is of advantage to add to the compositions of the present invention at least one of the substances selected from the group comprising chromium, vitamin B12, A-, D-, E, -C-vitamins, niacin, biotin, thiamine, B2-, B5-, B6-vitamins and folic acid and trace elements, such as chromium, manganese, selenium, zink and iron.

However, only those acetaldehyde-binding compounds, which are non-toxic and suitable for human consumption, can be applied to the compositions according to the present invention. These compounds should not cause a health hazard in the amounts used.

The most preferred compound is L-cysteine and the salts thereof.

The composition of the invention thus comprises one or more such acetaldehyde-binding compound(s), selected from L-cysteine and the salts thereof.

Useful compound to be added to the composition of the invention and for binding acetaldehyde is also lecithin.

A "harmful/carcinogenic content of acetaldehyde" in the human mouth, oesophagus, stomach, small intestine and large intestine is 20 to 800 µmol/1 of saliva or the contents of the intestine, a content of as low as about 20 to 50 µM causing carcinogenic mutations on the cell level. Hence, it would be advisable to aim at a zero concentration of acetaldehyde in these areas.

Keeping the acetaldehyde content essentially lower than without the use of the composition means that the acetaldehyde content should be kept at a level that is at least 20%, preferably over 40%, and most preferably over 60% lower than when not using the composition according to the description of the invention.

Such a harmful or carcinogenic content of acetaldehyde in the human mouth, oesophagus, stomach or small intestine or large intestine can be obtained in connection with consuming alcoholic drinks, particularly strong alcoholic drinks, or foodstuffs containing alcohol, as a consequence of smoking, when consuming products containing acetaldehyde and in particular in people having atrophic gastritis or achlorhydric stomach.

"Alcoholic drinks" are ethanol-containing drinks, their ethanol content varying within 0.7% by volume and 84% by volume."

"Alcoholic foodstuffs" refer to foodstuffs containing at least 0.7% of ethanol. Such foodstuffs can be, for example, fermented juices or preserves, or foodstuffs preserved with small amounts of alcohol, pastries, jellies, and mousse seasoned with liqueur or corresponding products containing alcohol.

"Acetaldehyde comprising foodstuffs" refers to foodstuffs containing acetaldehyde. Acetaldehyde is contained in foodstuffs, which have ethanol that is generated in connection with fermentation, such as beer, cider, wine, home-brewed beer, and other alcoholic drinks, as well as many juices. In certain foodstuffs, such as some milk products, acetaldehyde is used for preservation purposes and to add flavour, or the acetaldehyde is formed in the product as a consequence of microbial activity. For example, sugary juices or sugar-containing foodstuffs, in general provide a food substrate for microbes. High concentrations of acetaldehyde are formed, for example, in fermented milk products, such as yoghurt. The microbes used to make yoghurt produce acetaldehyde in the yoghurt. As for alcoholic drinks, sherry and Calvados contain especially large amounts of acetaldehyde.

The use of the compositions according to the invention can be of benefit even, when light alcoholic drinks are enjoyed or foodstuffs are consumed, which contain small amounts of alcohol.

"In connection with consuming alcoholic drinks" herein refers to the period of time that begins from starting to enjoy alcohol and ends, when there is no more alcohol in the blood.

"In connection with smoking" herein refers to the period of time that begins from starting to smoke and ends, when smoking is stopped.

"In connection with eating" herein refers to the period of time before, during and after eating.

According to one preferred embodiment of the invention the composition of the present invention is administered to people having an increased risk of developing cancer in the stomach. The acetaldehyde present on these areas can be locally bound by using the composition according to the invention into a harmless form by consuming the said compositions during or after eating.

Furthermore, according to the invention the efficiency of drug treatment can be improved by using the principles of theranostics. The key of theranostics (therapy specific diagnostics) is to improve the efficiency of drug treatment by helping physicians to identify patients who are the best candidates for the treatment in question. In addition, the adoption of theranostics could very well eliminate the unnecessary treatment of patients for whom therapy is not appropriate, resulting in significant drug cost savings for these patients.

According to one preferred embodiment of the invention the composition of the present invention is administered to people having atrophic gastritis or achlorhydric stomach.

In particular, the composition of the present invention is administered to people having a value outside the reference range or cut-off values of at least one of the biomarkers of atrophic gastritis selected from the group comprising pepsinogen I, pepsinogen II, pepsinogen I/pepsinogen II ratio and gastrin- 17 B (fast) and gastrin- 17S (stimulated). Also high HPAB (*Helicobacter pylori* antibody) value indicates a risk for developing atrophic gastritis. A suitable method and kit for examining the biomarkers is the commercially available GastroPanel® examination and software supporting its use (www.biohit.com/gastropanel , www.biohit.com/gastrosoft). The screening for atrophy of the corpus, mucosa of the whole stomach and antrum is described in US 6,696,262.

The GastroPanel® examination measures four biomarkers in blood: Pepsinogen I and II, Gastrin-17 and *Helicobacter pylori* antibodies. The GastroPanel® examination and the GastroSoft® software interpreting its results have been developed for use as a primary and follow-up examination in the diagnosis and treatment of patients with dyspepsia, *Helicobacter pylori* infection and atrophic gastritis and related risks (gastric cancer, vitamin B12 deficiency and peptic ulcer disease). Patients screened for having values outside the reference range or cut-off values of Pepsinogen I and II, gastrin-17 values and *Helicobacter pylori* antibodies and therefore having or having risk for developing atrophic gastritis (see Table 1) are treated by administering them the composition comprising acetaldehyde-binding substances of the present invention.

If the GastroPanel® examination gives a normal result, the diagnosis is either functional dyspepsia or another disease not involving the gastric mucosa. The examination diagnoses *Helicobacter pylori* infection, atrophic gastritis and its location (corpus, antrum or both). In addition to these diagnoses, GastroSoft® software also alerts to the risks associated with atrophic gastritis of the corpus of the stomach (gastric cancer and vitamin B12 deficiency) and to the risks associated with atrophic gastritis of the antrum (gastric cancer and peptic ulcer disease). The GastroSoft® report also indicates the risk of gastroesophageal reflux disease. If necessary, the report recommends further examinations, such as gastroscopy and biopsy specimen examination as well as vitamin B12 and homocysteine determinations (see Table 2).

The reference range for pepsinogen I value is between 30 - 120 µg/l, the reference range for pepsinogen II is 3 - 10 µg/l, the reference range for PGI/PGII ratio is 3- 20, and the reference range for Gastrin 17S (stimulated) value is 5 - 30 pmol/l, the reference range for Gastrin 17B (fast) is 2 - 10 pmol/l and the reference range for HPAB is 0 - 30 EIU.

Typical cut-off values for the biomarkers are selected from the group comprising: pepsinogen I 30 µg/l, pepsinogen II 3 µg/l, PGI/PGII ratio 3, Gastrin-17S (stimulated) value 5 pmol/l, Gastrin-17B (fast) 2 pmol/l and HPAB 30 EIU.

If pepsinogen I value is low, it is close to the lower limit or below the reference range 30 - 120 µg/l. Close to means typically lower limit +/-5. If pepsinogen II value is low, it is close to the lower limit or below the reference range 3 - 10 µg/l. Close to means typically lower limit +/-1. If PGI/PGII ratio is low, it is close to the lower limit or below the reference range 3- 20. Close to means typically lower limit +/-0.5. If Gastrin 17B (fast) value is high, it is close to the upper limit or above the reference range is 2 - 10 pmol/l. Close to means typically upper limit +/-0.5. If Gastrin-17S (stimulated) value is low, it is close to the lower limit or below the reference range is 5 - 30 pmol/l. Close to means typically lower limit +/-2. If HFAB is high it is close to or above the upper limit of the reference value 0-30 EIU. Close to means typically upper limit +/-3.

Pepsinogen I and II and their ratio act as biomarkers for atrophic gastritis in the corpus of the stomach. Five out of the seven early stages of the enzyme pepsin form the pepsinogen I group, which is produced only by the main cells in the corpus of the stomach and the mucus-secreting cells in the neck of the stomach. The remaining two form the pepsinogen II group, which is produced in the glands of the entire stomach and to some extent also in the Brunner glands in the upper duodenum. The lower the concentration of pepsinogen I detected in the plasma sample (reference range 30 - 120 µg/l) and/or the pepsinogen I to II ratio (the reference value being more than 3.0), the more severe the atrophic gastritis (Zagari et al. 2002, Sipponen et al. 2001, 2002, Väänänen et al. 2003, Pasechnikov et al. 2005, Nurgalieva et al. 2005, DiMario et al. 2005). Corpus atrophy increases the risk of gastric cancer of the corpus (Varis et al. 2000, Uemura et al. 2001, Zagari et al. 2002 and may result in vitamin B12 deficiency (Sipponen et al. 2003). An asymptomatic, progressive vitamin B12 deficiency of a few years' duration may cause permanent damage to the central and peripheral nervous system, resulting in e.g. dementia, depression and polyneuropathies. Vitamin B12 deficiency may also increase the concentration of homocysteine in the body, which has been thought to be an independent risk factor for atherosclerosis, strokes and cardiac attacks.

One component of gastric juice is hydrochloric acid (HCl), the secretory product of the parietal, or oxyntic cell of the corpus of the stomach. It is known that the capacity of the stomach to secrete HCl is almost linearly related to parietal cell numbers (Yao et al. 2003, Samuelson et al. 2003). Acid secretion is dependent on function of the H+/K+ ATPase or proton pump located in the cannilicular membrane of the parietal cell. Several drugs have been developed that non-competively bind and inactivate the ATPase, resulting in strong inhibition of acid secretion. Omeprazole (Prilosec) is an acid-activated prodrug that binds covalently to two cysteines on the ATPase, resulting in its irreversible inactivation. Other proton pump inhibitors (PPIs), including lansoprazole (Prevacid), esomeprazole (Nexium), rabeprazole (Aciphex) and pantoprazole (Protonix) have similar modes of action (Hellstrom et al.2004, Sachs et al 1994, Shamburek et al. 1992, Welag et al. 2003). The presence of gastrin stimulates parietal cells of the stomach to secrete hydrochloric acid (HCl) / gastric acid. Gastrin and hydrochloric acid form a known feed-back controlling mechanism that is an intimate part of the normal gastric physiology (Schubert 2004; Modlin et al. 1997).

Amidated gastrin-17, a peptide hormone, is the biomarker for atrophic gastritis of the antrum of the stomach. Physiologically, gastrin-17 is one of the most important fragments of gastrin. Gastrin (peptides consisting of 14, 17 and 34 amino acids) is formed in the G cells. G cells are found in the glandular epithelium of the antrum of the stomach and the duodenal mucosa. The blood concentration of amidated gastrin-17 produced by the G cells in the antrum continues to decrease as the atrophy of the antrum becomes more severe (Zagari et al. 2002, Väänänen et al. 2003, Pasechnikov et al. 2005, Sipponen et al. 2001, 2003, Nurgalieva et al. 2005, DiMario et al. 2005). If the patient has atrophic gastritis of the antrum of the stomach caused by a *Helicobacter pylori* infection, the fasting values of gastrin-17 are low (less than 2 pmol/l). In this case, the number of gastrin-17-secreting G cells in the mucosa of the stomach is decreased or the cells have disappeared completely (severe atrophy). The fasting value may also decrease if acid secretion in the stomach is high.

Gastric acid (HCl) inhibits the secretion of gastrin-17 from the G cells of the antrum, resulting in a reduced concentration of gastrin-17 in the plasma. Patients without *Helicobacter pylori* infection and with gastrin-17 fasting values of less than 2.0 pmol/l may be at risk of esophageal reflux disease and its complication, Barrett's esophagus. This risk is significantly more likely if the fasting value of gastrin-17 is 1.0 pmol/l or lower (Sipponen 2005).

If necessary, possible atrophic gastritis of the antrum can be confirmed or excluded by determining the concentration of protein-stimulated gastrin-17 in plasma in addition to fasting GastroPanel tests. It is thus possible to distinguish patients with atrophic gastritis in the antrum from patients whose low fasting concentration of gastrin-17 is entirely due to a high secretion of acid. If the antrum is not atrophied, protein stimulation (see www.biohit.fi / Service laboratory / Sampling instructions or www.biohit.com / Diagnostics / Instructions for the Collection of Blood Samples for the GastroPanel Examination) increases the production of gastrin-17 in the antrum G-cells, thus increasing the amount of gastrin-17 in the blood (over 5,0 pmol/l). If the protein-stimulated gastrin-17 concentration is less than 5.0 pmol/l and the patient has a *Helicobacter pylori* infection, it is very likely that the patient has atrophy of the antrum mucosa and a consequent risk of gastric cancer and peptic ulcer disease.

US Patent No. 6,696,262 discloses a method for screening atrophic gastritis based on quantitatively measuring pepsinogen I and gastrin-17 concentrations. The patent discloses also an immunoassay for detecting the presence of *Helicobacter pylori* infection.

Interpretation of GastroPanel® paramaters can be carried out based on the expected range according to the general information provided by table 1 (for more information GastroSoft ®).

**Table 1. How GastroPanel paramaters tends to behave in different cases**

| | PGI | PGII | PGI/II | G17B | G17S | HPAB |
|---|---|---|---|---|---|---|
| Atrophic gastritis in corpus | low | | low | high | | |
| Atrophic gastritis in antrum | | | | low | low | high |
| Atrophic gastritis in antrum/corpus | low | low | | low | low | |
| Non-atrophic gastritis | | high | | | | |
| Non-atrophic gastritis, *H.pylori* infection | | high | | | | high |
| Gastroesophageal reflux disease (GERD) | | | | low | | |

Table 2. Summary of the data provided by the GastroPanel examination and the ¹³C- urea breath - or stool antigen test of the "test-and-treat" strategy to the doctor in charge. The stochastic GastroSoft program supplies a patient report and in consecutive examinations the graphs on the probabilities of different conditions). The reports produced by GastroSoft are based on clinical studies comparing the results of GastroPanel examinations with results from gastroscopy and biopsy examinations (www.biohit.com/gastrosoft).

The serious medical and ethical problems of the test and treat strategy can be corrected simply and economically by replacing its ¹³C- urea breath - or stool antigen test by the GastroPanel examination (www.biohit.com/gastropanel).

| At an early stage... | The GastroSoft report states: | ¹³C - urea breath test or Stool antigen test report: |
|---|---|---|
| the diagnosis for | | |
| Functional vs. organic dyspepsia. | YES | NO |
| When GastroPanel indicates the gastric mucosa is healthy, the dyspepsia complaints are often caused by functional dyspepsia or another disease not involving the gastric mucosa | | |
| *H. pylori* infection (gastritis) | YES | NOT RELIABLE (1) |
| Atrophic gastritis (damaged and severely dysfunctional gastric mucosa) and the probabilities of different conditions affecting the mucosa of the gastric corpus or antrum or both (normal, gastritis or atrophic gastritis) | YES | NO |

| the risks (related to atrophic gastritis) of | | |
|---|---|---|
| Gastric cancer | YES | YES/NO (2) |
| Vitamin B12 deficiency | YES | NO |
| Peptic ulcer disease | YES | YES/NO (3) |

| the risks of | | |
|---|---|---|
| Gastroesophageal reflux disease and | YES | NO |
| Barrett's esophagus | YES | NO |

| if necessary, a recommendation for | | |
|---|---|---|
| Gastroscopy and biopsy examination | YES | NO |
| Treatment of *H. pylori* infection | YES | YES/NO (4) |
| Determination of vitamin B12 and homocysteine Follow-up examination to monitor | YES | NO |
| the incidence of atrophic gastritis | YES | NO |
| the healing of the *H. pylori* infection | YES | YES |
| the healing of atrophic gastritis | YES | NO |

| | | |
|---|---|---|
| (1) The ¹³C- urea breath - and stool antigen tests give false negative results if the patient has atrophic gastritis (a risk of gastric cancer and peptic ulcer disease and vitamin B12 deficiency and related diseases, such as dementia, depression and polyneuropathia as well as atherosclerosis, strokes and heart attacks), MALT lymphoma or bleeding peptic ulcer or if the patient is currently receiving antibiotics or PPIs. (2) The risk of gastric cancer is very low without atrophic gastritis in corpus, antrum or both. But in some cases, a *H. pylori* infection without histologically observable atrophic gastritis may be associated with gastric cancer and peptic ulcer disease. (3) No peptic ulcer disease with corpus atrophy (no acid, no ulcer). The risk of peptic ulcer disease is very low without antrum atrophy. (4) When the incidence of *H. pylori* -related atrophic gastritis is monitored, the patient can be offered targeted, safe treatment at the right time. The need for medication and the costs and adverse effects of medication can thus be reduced. If the patient has been diagnosed with peptic ulcer disease (gastric or duodenal ulcer), the *H. pylori* infection has to be treated (5). It should also be treated if the patient has atrophic gastritis. The patient and the doctor may also agree on eradication treatment for other reasons for example when the patient's close relatives have been diagnosed with gastric cancer. (5) Press Release: The 2005 Nobel Prize in Physiology or Medicine, 3 October 2005 jointly to Barry Marshall and J. Robin Warren for their discovery of "the bacterium Helicobacter pylori and its role in gastritis and peptic ulcer disease": - "An indiscriminate use of antibiotics to eradicate Helicobacter pylori also from healthy carriers would lead to severe problems with bacterial resistance against these important drugs. Therefore, treatment against Helicobacter pylori should be used restrictively in patients without documented gastric or duodenal ulcer disease." http://nobelprize.org/medicine/laureates/2005/press.html | | |

The diagnosis and treatment of atrophic gastritis of the stomach may be exemplified by the following example:

### Diagnosis and treatment or theranostics (i.e. therapy specific diagnostics)

In atrophic gastritis of corpus the concentration of Pepsinogen I and the ratio of the concentrations of Pepsinogen I/II decrease. In addition, since corpus does not secrete acid (HCl) due to atrophic gastritis because of the feed-back controlling mechanisms, the concentration of gastrin-17 (G-17) increases.

When the person has low pepsinogen I value, low pepsinogen I/ pepsinogen II ratio and high gastrin-17 (in particular G-17B fast) value, compared to the cut-off value or reference range, the person is diagnosed to have atrophic gastritis in the corpus, most often due to *Helicobacter pylori* infection and rarely due to autoimmune disease, leading to achlorhydric or low acid stomach and production of acetaldehyde by microbes in the stomach.

### Treatment

The composition comprising an effective amount of acetaldehyde-binding compound(s) is administered to the person. A suitable amount may be a composition comprising typically 100 - 200 mg L-cysteine in connection of eating, preferably two times a day, before, during or after eating.

The effect of the treatment may be monitored by testing later the concentrations of pepsinogen I, pepsinogen I/II, and Gastrin-17. A suitable time for monitoring may be 4 weeks and 8 weeks after the treatment was started or according to the estimate of the physician.

The diagnosis and testing can be made by commercially available GastroPanel® test kit.

### Achlorhydric stomach

Acid inhibitory drugs are widely used in Western societies, often long periods of time, and continuously in some patients. In various physiological experiments and small patient samples, acid inhibitory drugs are shown to influence serum gastrin, output of pepsin into gastric juice and output of pepsinogens into circulation (Gillen et al., 1999; Qvigstad & Waldum, 2004; Festen et al., 1984; Iwao et al., 1995; Brunner et al., 1995; Schumann &Massarat, 1991; Stoschus et al., 1998; Fraser et al., 1993; Ohsawa et al., 2002; Lazzaroni et al., 1992; Sanduleanu et al., 1999). According to our recent study it was examined how the use of antacids/alginates, H2RA's and PPI's influence the serum levels of gastrin-17 (G-17) and pepsinogens in everyday clinical practice.

PPI's, H2RA's and antacids/alginates affect acid secretion and intragastric acidity and may thereby influence normal gastric physiology. We examined the effect of these drugs on serum levels of gastrin-17 (G-17) and pepsinogens (PGI and PGII) in a large adult random population sample from Northern Sweden. The sample (n=1000, mean age 50.4 span 20-80) was endoscoped and biopsies were taken, and all subjects fulfilled a questionnaire of the use of acid inhibitory drugs (none; antacids/alginates; H2RA's or PPI's) during the last week or during the last three months. All subjects (n=590) with a normal gastric mucosa (no *Helicobacter pylori,* no gastritis nor atrophic gastritis) by biomarkers were analyzed for the influence of the acid inhibitory drugs on the fasting levels of serum G-17 B and PGI and PGII (and PGI/PGII ratio).

The serum levels of G-17 or pepsinogens did not differ between the users of antacids/alginates or H2RA and those who did not use the drugs. On the other hand, the mean and median levels of fasting serum G-17, PGI and PGII were significantly (P<0.001; non-parametric test) higher among the PPI users than among those without drugs. The mean and median levels of G-17, PGI and PGII were approximately doubled among the PPI users (both in those who used PPI during the last (previous) week or last three months) as compared to the means and medians in those without drugs. The ratio of PGI/PGII was, on the other hand, similar between the PPI users and those without drugs, or those with antacids/alginates or H2RA's. Among subjects with PPI use, the serum levels of pepsinogens correlated positively with the serum levels of G-17. The results are presented in Tables 3 and 4.

The PPI's but not the antacids/alginates or H2RA's raise markedly the fasting levels of serum G-17 and pepsinogens among ordinary users of these drugs. The increase in pepsinogens associates with an increase in G-17 among the PPI users.

The present findings support particularly the possibility that the action mechanisms of PPI's to raise serum pepsinogens are due merely to enhancement of the synthesis and/or release of pepsinogens from gastric corpus than to trophic influences alone. The serum levels of pepsinogens were approximately twice as high among the users of PPI as among the controls. This doubling of the serum pepsinogens cannot be hardly due to doubling of the mass of the oxyntic glands or cells in the stomach.

However, a long-lasting use of PPI's can cause parietal cell hypertrophy, and hypergastrinaemia of any reason may result in hyperplasia or hypertrophy of gastric corpus. Some earlier studies have shown that the Z-E syndrome is characterized by increased serum levels of both PGI and PGII (Lamers et al 1988, Biemond et al 1994) as was also the case in the present subjects who used PPI's.

**Table 3. Mean and median values of serum fasting gastrin-17 and pepsinogens in subjects with or without a use of acid inhibitory drugs during the last (previous) week.**

| | No drugs; Controls | Antacid | H2RA | PPI |
|---|---|---|---|---|
| | N=463 | 1 week N=29 | 1 week N=9 | 1 week N=23 |
| Gastrin-17 pmol/l | | | | |
| Mean ±S.D. | 5.0 ±11.3 | 3.8 ±3.4 | 3.0±3.3 | 24.7 ±66.4 *** |
| Median | 2.4 | 2.7 | 1.5 | 6.1 |

| Pepsinogen I (PGI) µg/l | | | | |
|---|---|---|---|---|
| Mean ±S.D. | 88.8 ±33.2 | 91.0±42.4 | 92.3 ±20.0 | 216 ±90.3*** |
| Median | 81.8 | 75.1 | 90.6 | 209 |

| Pepsinogen II (PGII) µg/l | | | | |
|---|---|---|---|---|
| Mean ±S.D. | 7.2 ±3.6 | 8.5 ±7.1 | 6.7 ±1.9 | 14.5 ±7.5*** |
| Median | 6.4 | 6.6 | 7.5 | 12.3 |

| PGI / PGII Ratio | | | | |
|---|---|---|---|---|
| Mean ±S.D. | 13.4 ±4.0 | 12.4 ±3.9 | 14.6 ±4.1 | 16.1 ±5.0** |
| Median | 13.3 | 12.5 | 14.6 | 16.0 |

| | | | | |
|---|---|---|---|---|
| difference significant ***(P<0.001), **(P<0.01) if compared with controls. Nonparametric test (Mann-Whithey U). | | | | |

**Table 4. Mean and median values of serum fasting gastrin-17 and pepsinogens in subjects with or without a use of acid inhibitory drugs during the last (previous) three months.**

| | No drugs; Controls | Antacid | H2RA | PPI |
|---|---|---|---|---|
| | N=419 | 3 months N=62 | 3 months N=16 | 3 months N=29 |
| Gastrin-17 pmol/l | | | | |
| Mean ±S.D. | 4.8 ±9.1 | 6.1 ±19.7 | 14.2 ±38 | 19.5 ± 59.7** |
| Median | 2.4 | 2.2 | 2.6 | 4.1 |

| Pepsinogen I (PGI) µg/l | | | | |
|---|---|---|---|---|
| Mean ±S.D. | 90.3 ±35.6 | 84.5 ±34.3 | 82.9 ±22.3 | 182 ±95.9*** |
| Median | 82.9 | 76.6 | 79.8 | 181 |

| Pepsinogen II (PGII) µg/l | | | | |
|---|---|---|---|---|
| Mean ±S.D. | 7.3 ±3.8 | 7.4 ±5.4 | 7.5 ±3.2 | 12.3 ±7.5*** |
| Median | 6.5 | 6.2 | 6.9 | 10.5 |

| PGI / PGII Ratio | | | | |
|---|---|---|---|---|
| Mean ±S.D. | 13.4 ±4.0 | 13.1 ±4.4 | 12.5 ±4.7 | 15.8 ±4.8** |
| Median | 13.3 | 12.6 | 13.0 | 15.9 |

| | | | | |
|---|---|---|---|---|
| difference significant ***(P<0.001), **(P<0.01) if compared with controls. Nonparametric test (Mann-Whitney U). | | | | |

### Diagnosis and treatment of achlorhydric or low acid stomach

In connection of PPI (Proton Pump Inhibitor) medication of gastroesophageal reflux disease or *Helicobacter pylori* infection the function of mucous membrane of stomach changes. This results mainly as increase of gastrin-17, in particular G-17 B (fast) concentration. The bigger decrease in the secretion of acid caused by PPI, the higher is the G-17 value. A strong increase in the concentration of G-17 indicates achlorhydric stomach, which remains as long as the PPI medication is continued. Also the PGI and PGII concentrations may increase, since direct PPI stimulus to the cells secreting PGI and PGII or/and the cells secreting try to cause corpus to secrete acid to non-acid stomach to compensate the situation.

Antacids and H2RA medications are used also for treatment of oesophageal reflux disease.

When the person has high gastrin-17 value, high PGI value and high PGII value as a result of PPI, antacid or H2RA medicaments, compared to the reference ranges in the population, the person is diagnosed to have achlorhydric or low acid stomach or a risk for it.

Achlorhydric or low acid (HCl) stomach is suitable environment for the growth of microbes of mouth. The microbes may produce ethyl alcohol and acetaldehyde from sugar and carbohydrate containing foodstuffs.

The composition comprising an effective amount of acetaldehyde-binding compound(s), such as the composition of the present invention, is administered to the person. A suitable amount may be a composition comprising typically 100 - 200 mg L-cysteine in connection of eating, preferably two times a day, before, during or after eating.

The effect of the treatment may be monitored by testing later the concentrations of PGI, PGII, and Gastrin-17. A suitable time for monitoring may be 4 and 8 weeks after the treatment was started.

The diagnosis and testing can be made by commercially available GastroPanel® test kit.

### Comparative compositions

Comparative compositions comprising acetaldehyde-binding compound(s) here mean also compositions intended for binding acetaldehyde (or aldehydes in general) in mouth. Such compositions can contain the acetaldehyde-binding compound(s) as described here and the compositions can be prepared as described in WO 2006/037848. Such compositions comprise chewable tablets, buccal tablets, sublingual tablets, candies, pastilles, lozenges, chewing gums and gels. Such preparations comprise preparations which release aldehyde-binding substances within less than 30 minutes, preferably within less than 15 minutes, typically within less than 10 minutes. Such preparations may comprise acetaldehyde (or aldehyde-binding) substances 1 to 500 mg, preferably 1 to 300 mg, still more preferably 1 to 250 mg, even more preferably 1 to 150 mg. According to a preferred embodiment the preparation may comprise 1 to 50mg, preferably 5 to 30 mg, more preferably 5 - 10 mg, typically 10 to 20 mg, suitable 1 to 5 mg or 1 - 20 mg.

By the composition comprising acetaldehyde-binding compound(s) is here meant also compositions intended for binding acetaldehyde (or aldehydes in general) in mouth as a long-acting preparation. Such preparations can contain the acetaldehyde-binding compound(s) as described here and the compositions can be prepared as described in WO 02/36098. Such compositions may comprise for example tablets or other preparations, which may be placed between the cheek or the lip and the gum, or preparations that are sucked or chewed in the mouth. Such preparations may comprise acetaldehyde (or aldehyde-binding) substances 1 to 500 mg, preferably 50 to 300 mg, more preferably 100 to 200 mg, still more preferably 1 to 250 mg, even more preferably 1 to 150 mg. According to a one preferred embodiment the preparation may comprise 1 to 50 mg, preferably 5 to 30 mg, more preferably 5 - 10 mg, typically 10 to 20 mg, suitable 1 to 5 mg or 1 - 20 mg. Under the oral conditions 15 to 25 mg of the compound(s) may be released in an hour.

### Other applications of the invention

If by the above described method, by using for example GastroPanel® test kit, is found *Helicobacter pylori* infection or a risk for gastroesophageal reflux disease, and if the patient has symptoms of it, it is of advantage, if PPI or other treatment decreasing the acidity of stomach, is combined with acetaldehyde-binding composition as a suitable preparation (e.g. capsule, tablet, granule, powder). By using the composition acetaldehyde produced by microbes in achlorhydric or low acid stomach, is bound into non-harmful acetaldehyde complex.

By the composition comprising acetaldehyde-binding compound(s) can be bound or inactivated acetaldehyde formed from ingested alcohol or from alcohol produced by microbes, and thereby decrease the disadvantages, such as cancer, risk for cancer and hangover. The composition is of particular advantage for those who have achlorhydric stomach or low acidity in stomach due to PPI or other treatment reducing the acidity of stomach or due to atrophic gastritis of corpus.

The composition comprising acetaldehyde-binding compound(s) can be used also in connection with metabolic syndrome. A person having metabolic syndrome typically has large waistline, high triglyceride level, high blood pressure, low level of (good) HDL cholesterol and high blood glucose level. Since the use of chromium as food supplement may be of benefit for a person having high blood sugar level, having high cholesterol level and fatness, it has been presented that one reason for metabolic syndrome may be the deficiency of chromium. Chromium is needed for the metabolism of carbohydrates and proteins. Chromium has an effect on the blood sugar level and reduces the desire for sweet. It is of advantage therefore to add chromium to the compositions of the present invention. It would have advantageous effect on metabolic syndrome and reduce the desire for sweet, which helps to control the weight and dieting.

Half of the people with atrophic gastritis of corpus of the stomach may have an exceptionally low vitamin B12 level and, of them, at least half have at the same time increased serum homocysteine. Vitamin B12 deficiency is a strong risk factor for neurodegenerative disorders, such as dementia, depression and polyneuropathies. The deficiency of vitamin B12 is one common reason for hyperhomocysteinemia, an independent risk factor for atherosclerosis, strokes and heart attacks. Because of corpus atrophy and poor diet even 15% of individuals over age 50 are suffering from a preventable epidemic of vitamin B12 deficiency. With early detection of corpus atrophy by the routine screening for the biomarkers, for example by GastroPanel® screening, and treatment, neurological disability (e.g. dementia, depression and polyneuropathies) and vascular diseases (e.g. stokes and heart attacks) can be prevented. The ageing of population is increasing the need for screening for the biomarkers, for example by the GastroPanel® examination and screening, and diagnosis of atrophic gastritis and related risks and diseases (see Table 2).

It is of advantage therefore to add vitamin B12 to the compositions of the present invention. By the compositions of the present invention can in addition to acetaldehyde-binding prevent the diseases following atrophic gastritis of corpus and having vitamin B12 deficiency, such as dementia, depression, polyneuropathies, arteriosclerosis and heart attacks and strokes.

To the compositions of the present invention including various preparations for gastrointestinal tract, and to the comparative compositions for the mouth, can be added in addition to chromium, vitamin B12 also other vitamins, such as A, D, E and C vitamins, niacin, biotin, thiamine, B2, B5, B6 and folic acid and trace elements, such as chromium, manganese, selenium, zink and iron. Ferrous compounds are of particular advantage, since atrophic gastritis is often connected with iron deficiency anaemia.

### Examples

**Comparative Example 1.** Gel forming formulation for prolonged binding of acetaldehyde in the stomach

The locally long-acting preparation that binds acetaldehyde in the stomach can be prepared and used to decrease the risk of cancer caused by acetaldehyde as follows:
The relative composition of the preparation that locally binds acetaldehyde in the stomach can be as follows, for example:

| | | |
|---|---|---|
| Cysteine | 60 | parts |
| Chitosan | 10 - 40 | parts |
| Calcium hydrogen phosphate | 0 - 30 | parts |

The powder mixture is mixed by conventional mixers (such as a blender), which are used in the pharmaceutical industry. After that, the powder mixture is granulated using a 2.5% acetic acid as a granulation liquid. The granulation liquid can be added to the same blender. The moist powder mass is pressed through a screen plate or a perforated plate (the diameter of the aperture being 2 mm). The formed granules are dried and screened. A screen fraction of 1.2-1.7 mm is recovered, which is dispensed into hard gelatine capsules so that the dose of cysteine is 100 mg. In the stomach gastric juice wet cysteine/chitosan granules forming a hydrogel. Gel starts to release cysteine in a prolonged way and reacts with acetaldehyde. Chitosan as gel forming component can be replaced by other well known gel forming pharmaceutical additives (e.g. alginic acid).

### Example 2. Non-disintegrating matrix tablet for binding acetaldehyde in the stomach)

The relative composition can be as follows:

| | |
|---|---|
| Cysteine | 25 parts |
| Eudragit RS | 20-30 parts |
| Microcrystalline cellulose | 20-50 parts |

From the powder mixture tablets containing 100 - 200 mg of cysteine can be compressed with equipments generally used in pharmaceutical industry. The tablet is a monolitic matrix tablet which does not disintegrate in the stomach. The active compound will release and dissolve in gastric fluid in a prolonged way leading to sustained acetaldehyde binding effect. Eudragit RS as a water insoluble binder can be replaced with similar pharmaceutical additives (e.g. ethylcellulose)

### Comparative Example 3. Film coated tablets for binding acetaldehyde in the stomach)

Pharmaceutical formulations releasing acetaldehyde-binding compound(s) in a sustained manner in the stomach can also be developed based on tablets coated with a porous film. The composition of the tablet core can be:

| | | |
|---|---|---|
| Cysteine | (20-50 parts) | 30 parts |
| Lactose | | 50-80 parts |
| Magnesium stearate | | 1-2 parts |
| Talcum | | 1-2 parts |

From the powder mixture tablets are compressed and film-coated utilizing techniques commonly used in pharmaceutical industry. The content of the coating solution can be, e.g.:

| | |
|---|---|
| Ethyl cellulose | 2-5 parts |
| Hydroxypropyl methylcellulose (HPMC) | 1-2 parts |
| Ethanol | 95 parts |

In the gastro-intestinal tract ethyl cellulose does not dissolve but HPMC dissolves forming pores to the film allowing cysteine to release from the tablet in a sustained manner.

### Example 4. Non-disintegrating granules for sustained release of acetaldehyde-binding substances in the stomach

The relative composition can be as follows, for example:

| | |
|---|---|
| Cysteine | 25 parts |
| Eudragit RS or ethylcellulose | 20-30 parts |
| Microcrystalline cellulose | 40-60 parts |
| Ethanol | q.s. |

Powdery substances are mixed and moistened by ethanol in equipments commonly used in pharmaceutical industry. The moistened mixture is granulated and dried by well-known methods. If required, the matrix granules formed can be coated with a low molecular weight hydroxypropyl methylcellulose film in order mask the taste of cysteine. Sufficient amounts of granules containing a single dose (100-200 mg) of cysteine can be dispensed into gelatine capsules or compressed with microcrystalline cellulose, e.g. to tablets.

### Example 5. Combination product for binding acetaldehyde in the stomach, intestine and colon

In practice it is quite often important to bind acetaldehyde in the stomach, intestine and colon at the same time. If a person suffers from achlorhydric or low acid stomach he or she most likely has acetaldehyde in the stomach. At the same time it is most likely that acetaldehyde can be found also in the intestine. If the person consumes alcoholic beverages it is obvious that acetaldehyde can be found also in the colon. For these reasons it is of advantage to develop a single formulation, which can release acetaldehyde-binding compound(s) and bind acetaldehyde in the whole length of the gastro-intestinal tract. For that purpose three different subformulations are incorporated to the same pharmaceutical product.

| | | |
|---|---|---|
| Fraction 1 | Equal to Example 4 | Release in the stomach |
| Fraction 2 | Consisting of Example 4 plus a film coat dissolving at pH 5-6 | Release in the intestine |
| Fraction 3 | Consisting of Example 4 plus a film coat dissolving at pH 6.5-7.5 | Release in the colon |

The relative amounts of fractions 1, 2 and 3 can be 2:1:1, 1:1:1, 1:1:2, 1:2:2, 1:2:3 or 1:1:3. Fraction 1 can be prepared as described in Example 4. Fractions 2 and 3 can be manufactured by film coating with well-known film-coating techniques. The film forming polymer for Fraction 2 could be Eudragit L, *e.g.,* and for Fraction 3 Eudragit S, *e.g.* The most convenient final product could be a hard gelatine or HPMC capsule. The total amount of cysteine in the combination product could be 200 to 500 mg.

### Example 6.

A composition comprising acetaldehyde-binding compound(s) was prepared as described in the earlier examples.

| | |
|---|---|
| L-cysteine | 25 w-% |
| Metacrylate polymer (Eudragit RS) | 25 w-% |
| Microchrystalline cellulose | 50 w-% |

The solid substances were mixed carefully in a suitable device. Ethanol was added in small amounts continuously mixing until a powder mixture having sufficient moisture was obtained. The moisture powder mixture was granulated by any in pharmaceutical industry commonly used method. The formed granules were dried.

The dissolution experiment was carried out by using an "artificial stomach". 25 ml of juice and yoghyrt contaminated with mouth bacteria were added to 100 ml bottles, which were incubated one day at room temperature. A preparation comprising 250 mg of L-cysteine was added to the bottles and they were slowly shaken at 37°C.

It was found that within 2 hours the total amount of acetaldehyde formed from the substrate was bound into harmless form.

### Literature

Biemond J, Kreuning J, Jansen JB, Lamers CB. Serum pepsinogens in patients with gastric diseases or after gastric surgery. Scand J Gastroenterol 1994;29:238-42
Brunner G, Hell M, Hengels KJ, Hennig U, Fuchs W. Influence of lasoprazole on intragastric 25-hour pH, meal.-stimulated gastric acid secretion, and concentrations of gastrointestinal hormones and enzymes in serum and gastric juice in healthy volunteers. Digestion 1995;56:137-144
Di Mario F, Franze A, Cavallaro LG. Non-Invasive Diagnosis for Gastric Diseases. One Global Medicine s.r.l. 2004; 1-48, www.biohit.com / Literature / Dignostics; 2004 Books
DiMario F, Cavallaro LG, Liatopoulou A, ym. Accuracy of "serological gastric biopsy" in a cohort dyspeptic patients, Poster presentation at the DDW 2005, May 15-18, in Chigago, IL, USA
Festen HP, Thijs LC, Lamers CB, Jansen JM, Pals G, Frants RR, Defize J, Meuwissen SG. Effect of oral omeprazole on serum gastrin and serum pepsinogen I levels. Gastroenterology 1984;87:1030-1034
Fraser AG, Lam WM, Luk YW, Sercombe J, Sawyerr AM, Hudson M, Samloff IM, Pounder RE. Effect of ranitidine bismuth citrate on postprandial plasma gastrin and pepsinogens. Gut 1993;34:338-342
Färkkilä M, Miten dyspepsiaa tulisi hoitaa, Duodecim 2004; 120: 2537 - 42
Gatta L, Perna F, Ricci C, ym. Effect of proton pump inhibitors and antacid therapy on 13C urea breath test and stool test for Helicobacter pylori infection. Am J Gastroenterol 2004;99:823-829
Gillen D, Wirz AA, Ardill JE, McColl KE. Rebound hypersecretion after omeprazole and its relation to on-treatment acid suppression and Helicobacter pylori status. Gastroenterology 1999;117:513-4
Graham KS, Graham DY. Contemporary Diagnosis and Management of H. pylori - Associated Gastrointestinal Diseases, Published by Handbooks in Health Care Co, Newtown, Pennsylvania, USA, 2002
Graham DY, Opekun AR, Hammoud F, Yamaoka Y, Reddy R, Osato MS, El-Zimaity HM. Studies regarding the mechanism of false negative urea breath tests with proton pump inhibitors. Am J Gastroenterol. 2003;98:1005-9.
Hellstrom PM, Vitols S: The choice of proton pump inhibitor: does it matter? Basic Clin Pharmacol Toxicol 94:105, 2004.
Iwao T, Toyonaga A, Kuboyama S, Tanikawa K. Effects of omeprazole and lanzoprazole on fasting and postprandial serum gastrin and serum pepsinogen A and C. Hepatogastroenterology 1995;42:677-682
Jauhonen P. Kajaanin dyspepsiatutkimuksesta henkilökohtainen tiedonanto 2005
Järvinen L. Tapausselostus: GastroPanel - uusi ase dyspepsian turvallisen hoidon kehittämiseen, Yksityislääkäri 2005; 2: 94 - 98
Kokkola A, Rautelin H, Puolakkainen P, ym. Positive result in serology indicates active Helicobacter pylori-infection in patients with atrophic gastritis. J Clin Microbiol 1998; 36 (6):1808-10148.
Kokkola A, Rautelin H, Puolakkainen P ym. Diagnosis of Helicobacter pylori- infection in Patients with Atrophic Gastritis: Comparison of Histology, 13C Urea Breath Test, and serology. Scand J Gastroenterol 2000; 25:138-141
Lamers CB, Rotter JI, Fansen JB, Samloff IM. Serum pepsinogen I in familial multiple endocrine neoplasia type I. Dig Dis Sci 1988;33:1274-6
Lazzaroni M, Sangaletti O, Bianchi Porro G. Gastric acid secretion and plasma gastrin during short-term treatment with omeprazole and raniditine in duodenal ulcer patients. Hepatogastroenterology 1992;39:366-370
Ohsawa T, Hirata W, Higichi S. Effects of three H2-receptor antagonists (cimetidine, famotidine, ranitidine) on serum gastrin level. Int J Clin Pharmacol Res 2002;22:29-35 Sandeleanu S, Strindsberg M, Jonkers D, Hameeteman W, Biemond I, Lundqvist G, Lamers C, Stockbrugger RW. Serum gastrin and chromogranin A during medium- and long-term acid suppressive therapy: a case-control study. Aliment Pharmacol Ther 1999;13:145-153
Schumann KM, Massarrat S. Changes in total pepsin activity and pepsinogen I in human sera under stimulation and inhibition of gastric acid secretion. Hepatogastroenterology 1991;38 Suppl 1:33-36
Stoschus B, Hamscher G, Ikonomou S, Partoulas G, Eberle C, Sauerbruch T, Feurle GE. Effect of omeprazole treatment on plasma concentrations of the gastric peptides, xenin, gastrin and somatostatin, and of pepsinogen. J Pept Res 1998;52:27-33
Manes G, Menchise A, deNucci C. Empirical prescribing for dyspepsia: a randomised controlled trial of test and treat versus omeprazole treatment. BMJ 2003;326:1118 - 1123
Modlin IM, Kidd M, Marks IN, Tang LH. The pivotal role of John S. Edkins in the discovery of gastrin. World J Surg 1997;21:226-234
Nurgalieva Z, El-Zimaity H, Graham D, ym. Gastric atrophyt in North America: Histology vs. Non-invasive testing, Poster presentation at the DDW 2005, May 15-18, in Chigago, IL, USA
Pasechnikov VD, Chukov SZ, Kotelevets SM, ym. Invasive and non-invasive diagnosis of Helicobacter pylori-associated atrophic gastritis: A comparative study, Scand J Gastroenterol 2005; 40: 297-301
Qvigstad G, Waldum H. Rebound hypersecretion after inhibition of gastric acid secretion. Basic Clin Pharmacol Toxicol 2004;94:202-8
Puustinen R. Helsingin kaupungin terveysasemien lääkäreiden käytettävissä olevia, "Käypä hoito"-suosituksen pikavalikoilta saatavia "gastroenterologisia tutkimuksia", henkilökohtainen tiedonanto 2005
Rugge M, Correa P, Dixon MF. ym. Gastric mucosal atroohy: interobserver consistency using new criteria for classification and grading. Aliment Pharmacol Ther 2002;16:1-12
Sachs G, Prinz C, Loo D, etc: Gastric acid secretion: activation and inhibition. Yale J Biol Med 67:81-95, 1994.
Salaspuro M. Ovatko "testaa ja hoida"-strategian haitat hyötyä suuremmat, Duodecim 2005;8:852-853
Salaspuro M. Dyspepsian kaksiportaisen seulontamenetelmän markkinointi on ennenaikaista, Duodecim 2005;121:1191-3
Samuelson LC, Hinkle KL: Insights into the regulation of gastric acid secretion through analysis of genetically engineered mice. Annu Rev Physiol 65:383-400, 2003.
Schubert ML. Gastric secretion. Curr Opin Gastroenterol 2004;20:519-25
Shamburek RD, Schubert ML: Control of gastric acid secretion. Histamine H2-receptor antagonists and
H+K(+)-ATPase inhibitors. Gastroenterology Clinics of North America. 21:527-550, 1992.
Sipponen P, Härkönen M, Alanko A. Atrofisen gastriitin toteaminen verinäytteestä. Suomen Lääkärilehti 2001; 38: 3833 - 3839
Sipponen P, Ranta P, Helske T, ym. Serum Levels of Amidated Gastrin-17 and Pepsino gen I in Atrophic Gastritis: An Observation Case-Control Study, Scand J Gastroenterol 2002 (7): 785 -
Sipponen P, Laxen F, Huotari K, ym. Prevalence of Low Vitamin B12 and High Homocysteine in Serum in an Elderly Male Population: Association with Atrophic Gastritis and Helicobacter pylori infection, Scand J Gastroenterol 2003; 12: 1209 -
Sipponen P, Vauhkonen M, Helske T, ym. Patients with Barrett's esophagus show low circulating levels of gastrin-17, World Gastroenterol 2005, in press
Talley NJ, Vakil N, Delaney G, ym. Management issues in dyspepsia: current consensus and controversies. Scand J Gastroenterol 2004; 39 (10): 913-918
Uemura N, Okamoto S, Yamamoto S, ym. Helicobacter pylori infection and the development of gastrici cancer, N Eng J Med 2001; 345:784-789
Varis K, Sipponen P, Laxen F ym. the Helsinki Gastritis Study Group,Implications of serum pepsinogen I in early endoscopic diagnosis of gastric cancer and dysplasia, Scand J Gastroenterol 2000; 9: 950-956
Väänänen H, Vauhkonen M, Helske T, ym. Non-Endoscopic Diagnosis of Atrophic Gastritis with a Blood Test. Correlation between Gastric Histology and Serum Levels of Gastrin-17 and Pepsinogen I. A Multicenter Study. Eur J Gastroenterol Hepatol 2003; 15: 885-891
Waldum HL, Brenna E, Sandvik AK. Long-term safety of proton pump inhibitors: risks of gastric neoplasia and infections. Expert Opin Drug Saf 2002;1 :29-38
Welage LS: Pharmacologic properties of proton pump inhibitors. Pharmacotherapy 23:74S-80S, 2003.
Whiting JL, Sigurdsson A, Rowlands DC, ym.The long term results of endoscopic surveillance of premalignant lesions. Gut 2002;50:378-81.
Wyeth Uutiset lääkäreille. Kaksiportainen menetelmä seuloo dyspepsiaa sairarastavat. Wyeth 2005;4.
Zagari RM, Nicolini G, Casanova S, ym. Diagnosis of atrophic gastritis in the general population based upon a combination of three non invasive tests, Gut 2002; 51 (suppl 11): A39.
Yao X, Forte JG: Cell biology of acid secretion by the parietal cell. Annu Rev Physiol 65:103-131, 2003.

## Claims

1. A non-toxic composition for decreasing the risk of developing cancer in the gastrointestinal tract of a person having atrophic gastritis or achlorhydric or low acid stomach, the risk being caused by acetaldehyde, the composition being in the form of a monolithic or multiparticular preparation which binds acetaldehyde present in the stomach or in the stomach, intestine and/or colon, wherein said composition comprises 1 to 40 w % of one or more acetaldehyde-binding compound(s), selected from L-cysteine and the salts thereof, wherein said compound(s) are mixed with a non-toxic carrier, being a polymer selected from methacrylate polymers and ethyl cellulose, and wherein the composition comprises 10-50 w % of polymer, and 20-70 w-% of a bulking agent, selected from calcium hydrogen phosphate and microcrystalline cellulose.

2. The composition according to claim 1, wherein the non-toxic carrier is selected from the metacrylate polymers Eudragit RS or S, or from ethyl cellulose.

3. The composition according to claim 1 or 2, wherein the composition comprises polymer additives that form a gel in the stomach, such as chitosans, alginates, sodium carboxy-methylcellulose grades, carbomers or aluminium hydroxide.

4. The composition according to any one of claims 1 to 3, wherein the composition comprises additives that render it fixable to the mucous membrane of the stomach, the additives being selected from cationic polymers, such as various chitosan grades.

5. The composition according to any one of claims 1 to 4, wherein the composition comprises additives that provide a preparation that floats in the stomach, the additives selected from polymers, such as alginic acid with added sodium hydrogen carbonate, or sodium alginate with aluminium hydroxide, sodium hydrogen carbonate, and water.

6. The composition according to claim 1, wherein the composition comprises bulking agents 40 to 60 w-%, most preferably about 50 w-%.

7. The composition according to any one of claims 1 to 6, wherein the composition is in the form of a matrix tablet or matrix granule.

8. The composition according to any one of claims 1 to 7, wherein the composition is coated with a water-soluble film, such as hydroxypropylmethylcellulose (HPMC) film, or it may be within a hard gelatine or HPMC capsule or tablet or other form of preparation.

9. The composition according to any one of claims 1 to 8, wherein the composition comprises porous film forming agents, such as ethyl cellulose and hydroxypropyl methyl cellulose.

10. The composition according to any one of claims 1 to 9, which is in the form of a preparation covered by a porous film, prepared from a water-soluble polymer, hydroxypropyl methyl cellulose (HPMC), and a water-insoluble polymer, ethyl cellulose (EC).

11. The composition according to claim 10, wherein the relative amount of EC / HPMC is 3/2 to 7/3.

12. The composition according to any preceding claim, which is in the form of a tablet or granule preparation, wherein the acetaldehyde-binding substance, mixed with fillers, has been granulated by using enteric polymers as binders, the enteric polymers preferably being selected from polymers with a solution pH of 6-7, and most preferably from Eudragit L and Eudragit S.

13. The composition according to claim 12, wherein the amount of enteric polymer is 2-5%, preferably 3-4%.

14. The composition according to any one of the preceding claims, wherein the composition comprises 1-500 mg, preferably 10-300, more preferably 100-200 mg of acetaldehyde-binding substance per single dose.

15. The composition according to any one of the preceding claims, wherein the compound(s) are released in the stomach for at least 30 minutes.

## Patentansprüche

1. Nichttoxische Zusammensetzung zum Senken des Krebsentwicklungsrisikos im Magen-Darm-Trakt einer Person mit atrophischer Gastritis oder achlorhydrischem oder geringsaurem Magen, wobei das Risiko durch Acetaldehyd verursacht wird, wobei die Zusammensetzung in der Form einer monolithischen oder multipartikulären Zubereitung ist, die Acetaldehyd, das im Magen, oder im Magen, Darm und/oder Dickdarm vorhanden ist, bindet, wobei die Zusammensetzung 1 bis 40 Gew.-% einer oder mehrerer Acetaldehyd-bindender Verbindung(en) umfasst, ausgewählt aus L-Cystein und den Salzen davon, wobei die Verbindung(en) mit einem nichttoxischen Träger vermengt werden, der ein Polymer ist, ausgewählt aus Methacrylatpolymeren und Ethylcellulose, und wobei die Zusammensetzung 10-50 Gew.-% Polymer und 20-70 Gew.-% eines Füllstoffs umfasst, ausgewählt aus Calciumhydrogenphosphat und mikrokristalliner Cellulose.

2. Zusammensetzung nach Anspruch 1, wobei der nichttoxische Träger aus den Methacrylatpolymeren Eudragit RS oder S, oder aus Ethylcellulose ausgewählt ist.

3. Zusammensetzung nach Anspruch 1 oder 2, wobei die Zusammensetzung Polymerzusätze umfasst, die ein Gel im Magen bilden, wie etwa Chitosane, Alginate, Natrium-Carboxymethylcellulosestufen, Carbomere oder Aluminiumhydroxid.

4. Zusammensetzung nach einem der Ansprüche 1 bis 3, wobei die Zusammensetzung Zusätze umfasst, die diese in die Lage versetzen, sich an der Magenschleimhaut festzusetzen, wobei die Zusätze aus kationischen Polymeren, wie etwa verschiedene Chitosanstufen, ausgewählt sind.

5. Zusammensetzung nach einem der Ansprüche 1 bis 4, wobei die Zusammensetzung Zusätze umfasst, die eine Zubereitung bereitstellen, die im Magen schwimmt, wobei die Zusätze aus Polymeren, wie etwa Alginsäure mit zugesetztem Natriumhydrogenkarbonat, oder Natriumalginat mit Aluminiumhydroxid, Natriumhydrogenkarbonat, und Wasser ausgewählt sind.

6. Zusammensetzung nach Anspruch 1, wobei die Zusammensetzung Füllstoffe 40 bis 60 Gew.-%, besonders bevorzugt ungefähr 50 Gew.-% umfasst.

7. Zusammensetzung nach einem der Ansprüche 1 bis 6, wobei die Zusammensetzung in der Form einer Matrixtablette oder eines Matrixgranulats ist.

8. Zusammensetzung nach einem der Ansprüche 1 bis 7, wobei die Zusammensetzung mit einem wasserlöslichen Film überzogen ist, wie etwa Hydroxypropylmethylcellulose (HPMC) -Film, oder sie kann innerhalb einer festen Gelatine oder HPMC-Kapsel oder Tablette oder anderen Zubereitungsform sein.

9. Zusammensetzung nach einem der Ansprüche 1 bis 8, wobei die Zusammensetzung poröse filmbildende Wirkstoffe, wie etwa Ethylcellulose und Hydroxypropylmethylcellulose umfasst.

10. Zusammensetzung nach einem der Ansprüche 1 bis 9, die in der Form einer mit einem porösen Film überzogenen Zubereitung ist, zubereitet aus einem wasserlöslichen Polymer, Hydroxypropylmethylcellulose (HPMC), und einem wasserunlöslichen Polymer, Ethylcellulose (EC).

11. Zusammensetzung nach Anspruch 10, wobei die relative Menge von EC/HPMC 3/2 bis 7/3 beträgt.

12. Zusammensetzung nach einem der vorstehenden Ansprüche, die in der Form einer Tabletten- oder Granulatzubereitung ist, wobei die Acetaldehyd-bindende Substanz, vermengt mit Füllmaterial, unter Verwendung von säureresistenten Polymeren als Bindemittel granuliert wurde, wobei die säureresistenten Polymere vorzugsweise aus Polymeren mit einem Lösungs-pH von 6-7, und besonders bevorzugt aus Eudragit L und Eudragit S ausgewählt sind.

13. Zusammensetzung nach Anspruch 12, wobei die Menge an säureresistentem Polymer 2-5 %, vorzugsweise 3-4 % beträgt.

14. Zusammensetzung nach einem der vorstehenden Ansprüche, wobei die Zusammensetzung 1-500 mg, vorzugsweise 10-300, besonders bevorzugt 100-200 mg Acetaldehyd-bindende Substanz pro Einzeldosis umfasst.

15. Zusammensetzung nach einem der vorstehenden Ansprüche, wobei die Verbindung(en) mindestens 30 Minuten lang im Magen freigesetzt werden.

## Revendications

1. Composition non toxique pour diminuer le risque de développer un cancer dans le tractus gastro-intestinal d'une personne ayant une gastrite atrophique ou un estomac achlorhydrique ou faiblement acide, le risque étant causé par de l'acétaldéhyde, la composition se présentant sous la forme d'une préparation monolithique ou multiparticulaire qui lie l'acétaldéhyde présent dans l'estomac ou dans l'estomac, l'intestin et/ou le côlon, dans laquelle ladite composition comprend 1 à 40% en poids d'un ou de plusieurs composés liant l'acétaldéhyde, sélectionnés à partir de L-cystéine et ses sels, dans laquelle ledit ou lesdits composés sont mélangés avec un support non toxique, étant un polymère sélectionné parmi les polymères de méthacrylate et l'éthylcellulose, et dans laquelle la composition comprend 10 à 50 % en poids de polymère et 20 à 70 % en poids d'un agent de charge, sélectionné à partir d'hydrogénophosphate de calcium et de cellulose microcristalline.

2. Composition selon la revendication 1, dans laquelle le support non toxique est sélectionné à partir des polymères métacrylates Eudragit RS ou S, ou à partir d'éthylcellulose.

3. Composition selon la revendication 1 ou 2, dans laquelle la composition comprend des additifs polymères qui forment un gel dans l'estomac, tels que des chitosanes, des alginates, des qualités de carboxy-méthylcellulose de sodium, des carbomères ou de l'hydroxyde d'aluminium.

4. Composition selon l'une quelconque des revendications 1 à 3, dans laquelle la composition comprend des additifs qui la rendent fixable à la membrane muqueuse de l'estomac, les additifs étant sélectionnés à partir de polymères cationiques, tels que diverses qualités de chitosane.

5. Composition selon l'une quelconque des revendications 1 à 4, dans laquelle la composition comprend des additifs qui fournissent une préparation qui flotte dans l'estomac, les additifs étant sélectionnés à partir de polymères, tels que l'acide alginique additionné d'hydrogénocarbonate de sodium, ou l'alginate de sodium avec de l'hydroxyde d'aluminium, de l'hydrogénocarbonate de sodium et de l'eau.

6. Composition selon la revendication 1, dans laquelle la composition comprend des agents de charge de 40 à 60 % en poids, le plus préférentiellement environ 50 % en poids.

7. Composition selon l'une quelconque des revendications 1 à 6, dans laquelle la composition est sous la forme d'un comprimé matriciel ou d'un granulé matriciel.

8. Composition selon l'une quelconque des revendications 1 à 7, dans laquelle la composition est enrobée d'un film hydrosoluble, tel qu'un film d'hydroxypropylméthylcellulose (HPMC), ou pouvant être contenue dans une gélatine dure ou une capsule ou un comprimé HPMC ou une autre forme de préparation.

9. Composition selon l'une quelconque des revendications 1 à 8, dans laquelle la composition comprend des agents filmogènes poreux, tels que l'éthylcellulose et l'hydroxypropylméthylcellulose.

10. Composition selon l'une quelconque des revendications 1 à 9, qui se présente sous la forme d'une préparation recouverte d'un film poreux, préparé à partir d'un polymère hydrosoluble, l'hydroxypropylméthylcellulose (HPMC) et d'un polymère insoluble dans l'eau, l'éthylcellulose (CE).

11. Composition selon la revendication 10, dans laquelle la quantité relative d'EC/HPMC est de 3/2 à 7/3.

12. Composition selon l'une quelconque des revendications précédentes, qui se présente sous la forme d'une préparation de comprimé ou de granule, dans laquelle la substance liant l'acétaldéhyde, mélangée à des charges, a été granulée en utilisant des polymères entériques comme liants, les polymères entériques étant de préférence sélectionnés à partir de polymères avec une solution pH de 6-7, et le plus préférentiellement à partir d'Eudragit L et d'Eudragit S.

13. Composition selon la revendication 12, dans laquelle la quantité de polymère entérique est de 2 à 5 %, de préférence de 3 à 4 %.

14. Composition selon l'une quelconque des revendications précédentes, dans laquelle la composition comprend 1 à 500 mg, de préférence 10 à 300, plus préférentiellement 100 à 200 mg de substance liant l'acétaldéhyde par dose unique.

15. Composition selon l'une quelconque des revendications précédentes, dans laquelle le ou les composés sont libérés dans l'estomac pendant au moins 30 minutes.
